# EUROPÄISCHE PATENTSCHRIFT

(11) **EP 2 814 414 B1**
(45) Veröffentlichungstag und Bekanntmachung des Hinweises auf die Patenterteilung: **21.11.2018**
(21) Anmeldenummer: 13703781.8
(22) Anmeldetag: 06.02.2013
(51) Int. Cl.: A61B 18/14

(54) **ELEKTROCHIRURGISCHES INSTRUMENT MIT EINEM BOGENFÖRMIGEN ELEKTRODENABSCHNITT**
ELECTROSURGICAL INSTRUMENT WITH AN ARCUATE ELECTRODE PART
INSTRUMENT ÉLECTROCHIRURGICAL AVEC UNE ÉLECTRODE POURVUE D'UNE PARTIE ARQUÉE

(30) Priorität: 16.02.2012 DE 102012101257
(43) Veröffentlichungstag der Anmeldung: 24.12.2014
(73) Patentinhaber: Aesculap AG, 78532 Tuttlingen (DE)
(72) Erfinder: ROTHWEILER, Christoph, 78166 Donaueschingen (DE); HERNER, Eugen, 72336 Balingen (DE); HUBER, Christian, 78570 Mühlheim (DE)
(74) Vertreter: Winter, Brandl, Fürniss, Hübner, Röss, Kaiser, Polte - Partnerschaft mbB
(86) Internationale Anmeldenummer: PCT/EP2013/052329
(87) Internationale Veröffentlichungsnummer: WO 2013/120743

(56) Entgegenhaltungen:
- WO-A2-2011/097469
- US-A- 3 920 021
- US-A- 6 123 701
- US-A1- 2004 019 352
- US-A1- 2006 217 709

## Beschreibung

Die vorliegende Erfindung betrifft ein elektrochirurgisches Instrument, insbesondere für laparaskopische Eingriffe, gemäß dem Oberbegriff des Anspruchs 1.

Nach chirurgischer Entfernung eines Hohlgefäßabschnitts, z.B. bei einer Darmresektion aufgrund eines mit einem Tumor befallenen Darmteils, müssen die zwei Hohlgefäßschnitte an ihren eröffneten Enden wieder so miteinander verbunden werden, dass ein kontinuierlicher Verlauf entsteht. Man spricht hierbei von einer End-zu-End-Anastomose. Standardmäßig werden hierbei die beiden eröffneten Enden z.B. mit Klammernahtgeräten wieder aneinander genäht.

Insbesondere bei Dünn- und Dickdarmeingriffen kommt es zuweilen zu undichten Nahtverbindungen (Nahtinsuffizienz), welche mit einem schweren Krankheitsverlauf und auch einer hohen Sterberate verbunden ist.

Eine Alternative zum Zusammennähen der Hohlgefäßabschnitte stellt die Thermofusions-Technik (TFT) dar. Die Thermofusion mittels Hochfrequenztechnik (HF) beruht auf der Denaturierung von Proteinen, die in vielen Geweben enthalten sind. Hierdurch ist es möglich, kollagenhaltige Gewebe zu verschweißen. Das Gewebe wird während des Schweißvorganges auf Temperaturen oberhalb der Eiweißdenaturierungstemperatur erhitzt und zusammen mit der intra- und extrazellulären Matrix in einen gelartigen Zustand gebracht. Nach Zusammendrücken der Gewebeflächen kühlt das verflüssigte Gewebe zu einer fusionierten Masse ab, was eine sichere Verbindung der Gewebe bewirkt.

Zum Verschweißen der Hohlgefäßschnitte wird das zwischen zwei Klemmbacken gefasste Gewebe mit einem Strom beaufschlagt, der zwischen Elektroden an den beiden Klemmbacken fließt.

Damit es nicht zum Versagen der Versiegelung bzw. der Verschweißung kommt, müssen die auf das Gewebe einwirkenden Parameter erfasst und geregelt werden. Um dies zu gewährleisten, benötigt man eine genaue Kontrolle von Temperatur, Druck, Gewebeimpedanz, Abstand und Lage.

Es ist wünschenswert, das zwischen den Klemmbacken gehaltene Gewebe gleichmäßig zu behandeln, so dass alle Bereiche zuverlässig erreicht werden und kein Bereich mit einem zu hohen Strom beaufschlagt wird. Dazu muss sichergestellt werden, dass die HF-Elektroden gleichmäßig voneinander beanstandet sind bzw. parallel zueinander ausgerichtet sind.

Aus dem Stand der Technik sind keine Instrumente geeigneter Größenordnung für die Anwendung an den oben genannten Hohlgefäßen und Gewebearten bekannt. Bei Koagulationsinstrumenten kleinerer Bauart, wie z.B. in EP 1 747 762 A2 gezeigt, kommt es, bedingt durch die Bauart, beim Schließen der Klemmbacken zu einer nicht parallelen Ausrichtung der HF-Elektroden.

Benachbarter Stand der Technik ist aus den Druckschriften DE 20 2004 009 427 U1, US 2006/0217709 und DE 699 29 230 T2 bekannt.

Der Abstand zwischen den Elektroden kann durch an den Klemmbacken angebrachte Abstandshalter eingehalten werden. Wenn jedoch auf den Klemmbacken eine größere Anzahl von Abstandshaltern vorgesehen ist, wie dies z.B. in EP 1 656 901 B1, EP1 952 777 A1, EP 1 372 507 A1 oder US 2004/122423 A1, gezeigt ist, perforieren die Abstandshalter zwangsläufig das zu behandelnde Gewebe, da das Gewebe unter den Abstandhaltern bei geschlossenen Klemmbacken derart komprimiert wird, dass es zu dauerhaften Gewebeschädigungen kommt. Dies hat negative Auswirkungen auf das Ergebnis der Versiegelung.

Wenn der Anpressdruck der Klemmbacken reduziert wird, um eine Perforation des Gewebes zu vermeiden, und das Gewebe unter den Abstandshaltern lediglich eingeklemmt wird, führt dies zu einer winkligen Auslenkung der Klemmbacken.

Da die Abstandshalter ferner aus elektrisch nicht leitendem Material sind, um einen Kurzschluss zwischen den HF-Elektroden zu vermeiden, entsteht im Bereich dieser Abstandshalter ein sog. Koagulationsschatten, d.h. dass die Gewebeabschnitte im Bereich der oder unter den Abstandshaltern abgekapselt sind, somit nicht oder nur ungenügend mit Strom beaufschlagt werden und es dort zu keiner zufriedenstellenden Verschweißung der Gefäßabschnitte kommt.

Wenn die Anzahl der Abstandshalter reduziert wird, vergrößert sich zwangsläufig der Abstand zwischen den einzelnen Abstandshaltern. Wenn die Klemmbacken mit hohem Anpressdruck gegeneinander gepresst werden, kommt es selbst bei der Verwendung steifer Materialien zwischen den Abstandshaltern zu Durchbiegungen der Klemmbacken und der Elektroden, so dass Abstand der Elektroden zwischen den Abstandshaltern kleiner als an den Abstandshaltern ist. Diese Unterschiede in den Abständen der Elektroden führt zu einer inhomogenen Durchdringung des Gewebes mit HF-Energie.

Vor diesem Hintergrund liegt der vorliegenden Erfindung die Aufgabe zugrunde, ein Instrument bereitzustellen, das mittels Thermofusions-Technik das Ergebnis einer End-zu-End-Anastomose von Hohlgefäßen wie Dünn- und Dickdarm bzw. allgemein bei Gewebeverbindungen verbessert, insbesondere einen gleichmäßigen Abstand von gegenüberliegenden HF-Elektroden ohne Schädigung des Gewebes sicherstellt.

Eine weitere Aufgabe der vorliegenden Erfindung ist die Bereitstellung von für das Instrument geeigneten Elektroden.

### Zusammenfassung der Erfindung

Die Aufgabe wird hinsichtlich des Instruments durch die Merkmale des Anspruchs 1 gelöst. Vorteilhafte Weiterbildungen sind Gegenstand der Unteransprüche.

Alle im Folgenden genannten Beispiele und Ausführungen, die nicht unter den unabhängigen Anspruch 1 fallen, sind nicht Teil der Erfindung.

Ein erfindungsgemäßes elektrochirurgisches Instrument hat gegeneinander bewegbare Instrumentenbranchen mit jeweils einer oder mehreren Elektroden(-flächen), zwischen welchen ein Gewebe eingeklemmt und elektrothermisch behandelt werden kann. Die scherenartige Bewegung der Instrumentenbranchen relativ zueinander wird in Schließrichtung durch zumindest zwei in Längserstreckung der Instrumentenbranchen von einander beabstandete und auf die Instrumentenbranchen wirkende Abstandshalter begrenzt. Erfindungsgemäß ist zumindest ein in einer Schließstellung der Instrumentenbranchen auf Durchbiegung beanspruchter Elektrodenflächenabschnitt entgegen der zu erwartenden Biegerichtung und in Längsrichtung bogenförmig, insbesondere in Richtung zur gegenüberliegenden Branche konkav, ausgebildet.

Durch die spezielle Form der Elektrode(n) kann gewährleistet werden, dass der Abstand der sich gegenüberliegenden Elektrodenflächen unter Berücksichtung der Biegebeanspruchung in der Schließstellung der Instrumentenbranchen über die gesamte Elektrodenlänge und -fläche im Wesentlichen gleichmäßig groß ist. Durch diese Gestaltungsform der Elektroden wird auch gewährleistet, dass es zu einer im Wesentlichen gleichbleibenden Flächenpressung des zu verbindenden Gewebes kommt. Hierdurch werden gleichbleibende Voraussetzungen für die HF-Chirurgie, insbesondere im Hinblick auf die Gewebeimpedanz geschaffen. Dadurch kann die Qualität der versiegelten Gewebebereiche besser elektrisch kontrolliert werden. Durch die spezielle Ausführung der Elektroden und der Abstandshalter werden Kurzschlüsse zwischen den Elektroden vermieden. Darüber hinaus kommt es durch die spezielle Anordnung zu einer homogenen Stromverteilung im Gewebe.

Durch die bogenförmige (bezüglich der Kontaktseite konkave) Krümmung der Elektrode(n) kann der Abstand zwischen den Abstandshaltern vergrößert werden und somit die Anzahl der Abstandshalter insgesamt verringert werden, ohne dabei Gefahr zu laufen, dass die Elektrodenflächen den Mindestabstand unterschreiten.

Wie eingangs bereits erwähnt, wird bei einer zu großen Anzahl von Abstandshaltern, insbesondere wenn diese auf den Elektrodenflächen angeordnet fixiert sind, das zu versiegelnde Gewebe zu stark vorgeschädigt. D.h., die (noppenförmigen) Abstandshalter würden das eingeklemmte Gewebe perforieren. Ferner entstehen viele Koagulationsschatten.

Das erfindungsgemäße elektrochirurgische Instrument bzw. die erfindungsgemäßen Elektroden eines solchen elektrochirurgischen Instruments schaffen einen optimalen Kompromiss zwischen maximaler paralleler Ausrichtung der HF-Elektroden einerseits und homogener Gewebefusion bei minimaler Gewebeschädigung anderseits.

Somit werden durch die Abstandshalter verursachte Gewebeschäden durch die überhöhte Krafteinwirkung auf das eingespannte Gewebe verhindert und eine sichere Fusion der einzelnen Gewebebestandteile durch gleich bleibende Kraftverhältnisse, durch die parallele Anordnung der Elektroden, durch den klar definierten Abstand der Elektroden und durch die homogene Stromverteilung im Gewebe gewährleistet. Durch die spezielle Form der Elektroden (d.h. zueinander konkav) werden Kurzschlüsse zwischen den Elektroden und Leckagen an den versiegelten Gewebeschichten insbesondere im Längsmittenbereich der Branchen vermieden werden.

Dadurch werden gleich bleibende Voraussetzungen für HF-Chirurgie insbesondere im Hinblick auf die Gewebeimpedanz geschaffen. Dadurch kann die Qualität der versiegelten Gewebebereiche besser elektrisch kontrolliert werden.

Insbesondere bei besonders langen Instrumentenbranchen oder solchen, die mittig an Betätigungshebeln gelagert sind, muss sichergestellt werden, dass die Elektrodenflächen im medialen Bereich den gewünschten Abstand halten und dieser aufgrund von Biegebeanspruchung nicht unterschritten wird. Aufgrund der Bogenform der Elektroden im Mittenabschnitt voneinander weg kann auf einen Abstandshalter, der auf die medialen Abschnitte der Instrumentenbranchen wirkt, verzichtet werden, wodurch die Gesamtzahl notwendiger (noppenförmiger) Abstandshalter verringerbar ist.

Gemäß einem weiteren oder unabhängigen Aspekt kann zumindest ein zwischen den zwei in Längserstreckung der Instrumentenbranchen von einander beabstandete Abstandshaltern angeordneter Elektrodenflächenabschnitt zumindest im Längsmittenabschnitt bogenförmig oder konkav ausgebildet sein.

Dies bedeutet vorzugsweise einerseits, dass die Elektrode insgesamt oder zumindest deren dem zu behandelnden Gewebe zugewandte Elektrodenfläche über ihre gesamte Längserstreckung (konkav) bogenförmig vorgeformt ist. Durch die bogenförmige Vorformung wirkt die Elektrode bei Zusammenpressen mit der gegenüberliegenden Elektrode wie eine Art Blattfeder, die durch den Anpressdruck der Instrumentenbranchen plan gedrückt wird und dadurch die gewünschte parallele Ausrichtung zu der anderen Elektrode erzielt.

Es ist jedoch ausreichend, lediglich diejenige Elektrodenflächenabschnitte (konkav) vorzuformen, die einer Biegebeanspruchung ausgesetzt sind, d.h. die Abschnitte zwischen zwei Auflagepunkten der Elektroden bzw. zwischen zwei Abstandshaltern.

Der Elektrodenflächenabschnitt kann entsprechend der in der Schließstellung der Instrumentenbranchen zu erwartenden Biegebeanspruchung derart vorgeformt sein, dass dieser in der Schließstellung der Instrumentenbranchen den durch die Abstandshalter definierten Mindestabstand zwischen den Elektrodenflächen nicht unterschreitet.

Dadurch kann sichergestellt werden, dass die Elektrodenflächen trotz der Biegebeanspruchung an keiner Stelle den für die gleichmäßige Stromdurchdringung wesentlichen Mindestabstand unterschreiten bzw. die Elektrodenflächen über die gesamte Länge einen im Wesentlichen gleichmäßigen Abstand zueinander haben.

Der Elektrodenflächenabschnitt kann entsprechend der in der Schließstellung der Instrumentenbranchen zu erwartenden Biegebeanspruchung derart vorgeformt sein, dass dieser in der Schließstellung der Instrumentenbranchen im Wesentlichen gerade gebogen wird.

Dadurch kann trotz großer Abstände zwischen den Abstandshaltern in der Schließstellung der Instrumentenbranchen eine parallele Ausrichtung der Elektrodenflächen zueinander gewährleistet werden.

Der Elektrodenflächenabschnitt kann entsprechend der Anzahl und der Abstände der Abstandshalter sowie entsprechend dem Elastizitätsmodul und Flachenträgheitsmoment der entsprechenden Instrumentenbranche vorgeformt sein.

Wenn die Instrumentenbranchen in der Schließstellung zum Greifen und Behandeln eines Gewebes gegeneinander gepresst werden, hängt die Biegebeanspruchung neben der Anzahl und den Abständen der Abstandshalter im Wesentlichen von den Material- und Geometrieeigenschaften der Instrumentenbranchen ab, die in der Regel biegesteifer als die daran angebrachten Elektroden sind. Vor diesem Hintergrund ist es sinnvoll, die Vorformung der Elektrodenflächen in Abhängigkeit der Eigenschaften der Instrumentenbranchen insgesamt zu bestimmen.

Anstatt lediglich die Elektrodenfläche bogenförmig zu gestalten, kann die gesamte Instrumentenbranche samt der Elektrodenfläche (konkav im Sinne vorstehender Beschreibung) bogenförmig ausgebildet sein.

Wenn die beiden Instrumentenbranchen ähnliche (biegeflexible) Eigenschaften aufweisen, können die einander gegenüberliegenden Elektrodenflächen der Instrumentenbranchen spiegelsymmetrisch ausgebildet sein. Dies erleichtert die Fertigung der Elektroden, da diese sowohl für die eine als auch für die anderen Instrumentenbranchen verwendet werden können.

Wenn die Abstandshalter ausschließlich auf proximale und distale Endabschnitte der Instrumentenbranchen wirken, kann der gesamte mediale Bereich der Elektrodenflächen (konkav) bogenförmig ausgebildet werden.

Dadurch kann der gesamte für die Behandlung des Gewebes wesentliche mediale Bereich der Elektroden komplett von Abstandshaltern freigehalten werden. Dadurch werden abstandshalterbedingte Gewebeschädigungen und Koagulationsschatten vermieden.

Der auf die distalen Endabschnitte der Instrumentenbranchen wirkende Abstandshalter kann durch einen an einem distalen Endabschnitt einer Instrumentenbranche zwischen zwei Elektrodenflächen angeordneten und zur anderen Instrumentenbranche hin weisenden Vorsprung oder durch einen auf einer Elektrodenfläche oder durch mehrere auf jeweils unterschiedlichen Elektrodenflächen vorgesehene (fixierte) Vorsprünge gebildet werden. Der auf die proximalen Endabschnitte der Instrumentenbranchen wirkende Abstandshalter kann durch ein separat von den Instrumentenbranchen ausgebildetes Abstandshaltermodul (Bauteil), das zumindest eine Materialzunge (Zungenplättchen) aufweist, welche in einer Schließstellung zwischen den Instrumentenbranchen einklemmt wird, oder durch ein Zusammenwirken zumindest eines an einer der Instrumentenbranchen vorgesehenen Drehbegrenzungsstifts und einer im Betätigungshebel ausgeformter Kulisse, in welcher der Drehbegrenzungsstift geführt ist, gebildet werden.

Durch diese speziellen Anordnungen der Abstandshalter können die Elektrodenflächen weitestgehend frei von Abstandshaltern gehalten werden, wodurch das Risiko von Gewebeschädigungen und Koagulationsschatten weiter minimiert werden kann.

Eine erfindungsgemäße Elektrode, insbesondere für ein oben beschriebenes elektrochirurgisches Instrument mit einer Gewebekontaktseite oder -fläche, ist zumindest abschnittsweise in Längsrichtung und entgegen einer zu erwartenden Biegerichtung bogenförmig oder konkav ausgebildet. Insbesondere kann die Elektrode insgesamt oder zumindest die für die elektrothermische Behandlung eines Gewebes vorgesehene Gewebekontaktseite oder -fläche der Elektrode zumindest abschnittsweise in Längsrichtung und senkrecht zur Gewebekontaktfläche bogenförmig, insbesondere konkav, ausgebildet sein.

Durch Verwendung einer erfindungsgemäßen Elektrode in einem elektrochirurgischen Instrument kann sichergestellt werden, dass durch deren spezielle (konkave) Vorformung zu erwartende Biegebeanspruchungen bei Zusammenpressen der Branchen ausgeglichen werden können und so gleichmäßige Abstände zwischen den Elektroden erzielt und Unterschreitungen von Mindestabständen und sogar Kurzschlüsse vermieden werden können.

Die Elektrode kann an einem distalen Endabschnitt und an einem proximalen Endabschnitt jeweils einen als Abstandshalter fungierenden Vorsprung, insbesondere aus nicht leitendem Material, aufweisen und die Elektrode zwischen den Vorsprüngen bogenförmig oder konkav ausgebildet sein.

Eine solche Elektrode stellt im eingebauten Zustand zum Einen sicher, dass zwei Instrumentenbranchen einen durch die Vorsprünge vorbestimmten Mindestabstand zu einander über die gesamte Branchenlänge wahren, und zum Anderen, dass der Mindestabstand trotz etwaiger Biegebeanspruchung auch zwischen den Abstandshaltern gewahrt bleibt, zumindest nicht unterschritten wird.

Zudem kann die Biegung oder Krümmung der Elektrode entgegen einer zu erwartenden Biegerichtung in Schließstellung verlaufen, sodass sich in Schließstellung ein im Wesentlichen gerader Elektrodenverlauf einstellt.

Es ist zu beachten, dass die zuvor genannten Aspekte und Merkmale sowohl einzeln, als auch zu mehreren miteinander kombiniert werden können.

### Kurze Beschreibung der Zeichnungen

Fig. 1 zeigt ein elektrochirurgisches Instrument gemäß einer ersten Ausführungsform der Erfindung
Fig. 2 zeigt eine perspektivische Ansicht zweier zueinander verschwenkbarer Instrumentenbranchen des elektrochirurgischen Instruments gemäß einer ersten Ausführungsform der Erfindung;
Fig. 3 zeigt eine Seitenansicht der beiden Instrumentenbranchen der Fig. 2 in einer geöffneten Stellung;
Fig. 4 zeigt eine (nicht maßstabsgetreue) Prinzipskizze eines gegenüberliegenden Paars von Elektroden des elektrochirurgischen Instruments gemäß einer ersten Ausführungsform der Erfindung, in einem unbelasteten und einem belasteten Zustand; und
Fig. 5 zeigt ein elektrochirurgisches Instrument gemäß einer sechsten Ausführungsform der Erfindung.

### Detaillierte Beschreibung bevorzugter Ausführungsformen

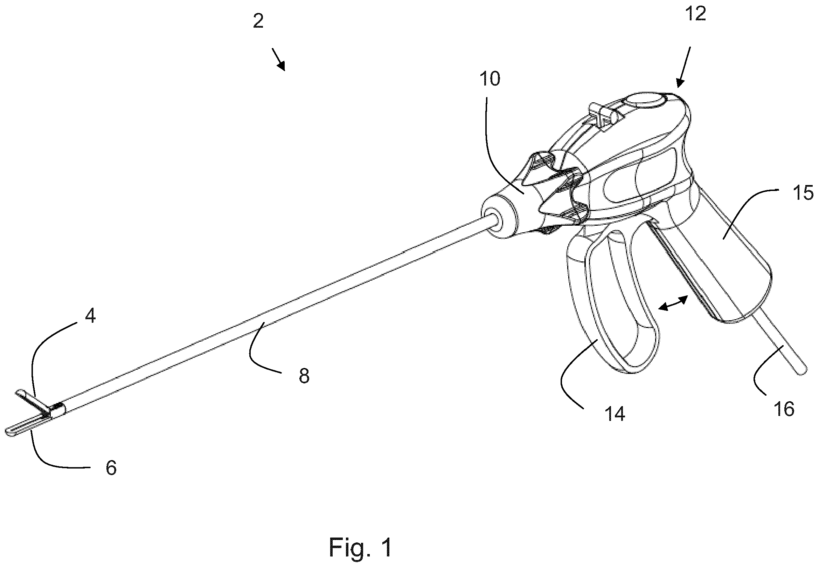

Fig. 1 zeigt eine perspektivische Ansicht eines laparaskopischen elektrochirurgischen Instruments bzw. einer Koagulationsklemme 2 gemäß einer ersten Ausführungsform der Erfindung mit einem Paar Instrumentenbranchen 4 und 6 in einer geöffneten Stellung, die am distalen Ende eines (Instrumenten-) Schafts 8 angeordnet sind, der wiederum über eine Schaftdreheinrichtung 10 an einem Handhabungsteil oder Griffstück 12 drehbar befestigt ist. Über die Schaftdreheinrichtung 10 kann der Schaft 8 und die daran angeordneten Instrumentenbranchen 4 und 6 relativ zum Handhabungsteil 12 um die Schaftlängsachse verdreht werden. Das Handhabungsteil 12 weist einen betätigbaren Handgriff oder Trigger 14 auf, der relativ zu einem zweiten mit dem Handhabungsteil 12 fest verbundenen Handgriff (Pistolengriff) 15 bewegbar ist. Die scherenartig sich bewegenden Instrumentenbranchen 4, 6 stehen über ein (nicht gezeigten) Betätigungsmechanismus, z.B. einem Seilzug oder eine Schubstange, in Wirkverbindung mit dem Handgriff 14 und können durch Betätigung des Handgriffs 14 stufenlos von einer geöffneten in eine geschlossene Stellung und umgekehrt gebracht werden. Über eine (nur zum Teil gezeigte) Leitung (oder ein elektrisches Stromkabel) 16 ist das Handhabungsteil 12 mit einer (nicht gezeigten) HF-Energiequelle verbunden, um zur elektrothermischen Behandlung von Gewebe zwischen den Instrumentenbranchen 4 und 6 eine HF-Spannung anlegen zu können.

Hinsichtlich der allgemeinen Funktionsweise und des mechanischen Aufbaus des Instruments 2 wird beispielsweise auf die veröffentlichte Druckschrift WO 2011/097469 A2 verwiesen.

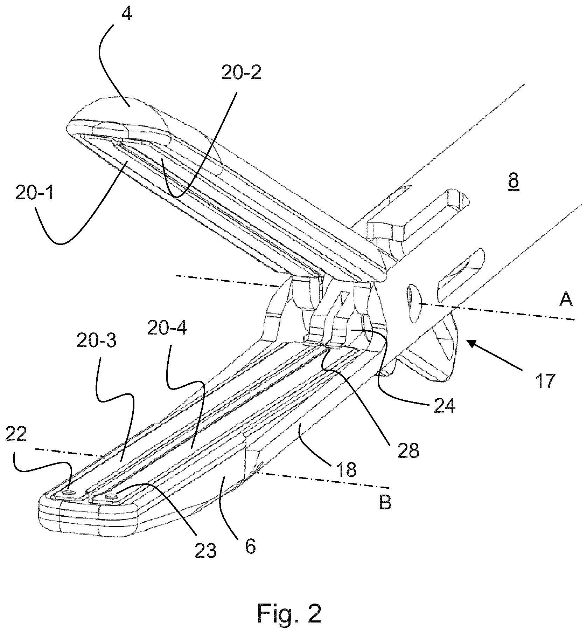

Fig. 2 zeigt lediglich das distale Ende des Schafts 12 bzw. das Instrumentenmaulteil 12 mit den Instrumentenbranchen 4 und 6 in einer geöffneten Stellung. Die erste bzw. gemäß Fig. 2 obere Instrumentenbranche 4 ist über ein proximales Schwenkgelenk 17 um eine Querachse A schwenkbar am distalen Ende des Schafts 12 gelagert. Die zweite bzw. untere Instrumentenbranche 6 ist zum Teil in einem schalenförmigen und über das Schwenkgelenk 17 hinaus vorspringenden Stütz-/Auflagerabschnitt 18 des Schafts 12 aufgenommen und mittig an diesem wippemförmig angelenkt. Über einen nicht gezeigten Federmechanismus (siehe WO 2011/097469 A2), ist die vordere (distale) Spitze der unteren Instrumentenbranche 6 nach oben bzw. zur oberen Instrumentenbranche 4 hin vorgespannt, um das Greifen von Gewebe zu erleichtern. Die untere Instrumentenbranche 6 ist nur soweit am Stützabschnitt 18 schwenkbar gelagert (Achse B), um kleinere Winkelabweichungen zwischen der oberen und unteren Instrumentenbranche 4 und 6 im zusammengepressten Zustand ausgleichen zu können.

Jede der Instrumentenbranchen 4 und 6 weist zwei in Querrichtung jeder Branche voneinander beabstandete und parallel längs verlaufende Elektrodenflächen 20 (20-1, 20-2, 20-3 und 20-4) auf, welche mit HF-Spannung beaufschlagt werden können. Befindet sich Gewebe zwischen den Instrumentenbranchen 4 und 6 in ihrer geschlossenen Stellung, kann der Chirurg dieses mit den Elektrodenflächen 20 koagulieren, trennen oder verschweißen. Zwischen den Elektrodenflächen 20 kann zusätzlich ein (nicht gezeigtes) spezielle elektrochirurgisches Messer oder eine Schneideeinrichtung angeordnet sein.

Um einen Kurzschluss zwischen den Elektrodenflächen 20 der beiden Instrumentenbranchen 4 und 6 zu vermeiden bzw. um sicherzustellen, dass ein in Längsrichtung homogener Strom über das zwischen den Elektrodenflächen eingeklemmte Gewebe fließt, müssen die Elektrodenflächen 20 auch in der geschlossenen Stellung vorzugsweise gleichmäßig voneinander beabstandet bleiben. Das Instrument 2 hat deshalb am distalen Abschnitt zumindest der unteren und/oder oberen Instrumentenbranche 6, (4) auf den beiden parallelen Elektrodenflächen 20-3 und 20-4 einen über die Elektrodenflächen 20-3 und 20-4 um ein vorbestimmtes und dem gewünschten Abstand zwischen den Elektrodenflächen 20 entsprechenden Maß hinaus vorspringende (noppenförmige) Vorsprünge 22 und 23, die mit den entsprechenden Elektrodenflächen 20-1 bzw. 20-2 der oberen Instrumentenbranche 4 in Anlage kommen und so als Abstandshalter an den distalen Abschnitten der beiden Instrumentenbranchen 4 und 6 dienen. Diese Vorsprünge 22, 23 können aufgeklebt, aufgelötet, aufgespritzt, oder auf sonstige geeignete Weise fest oder lösbar aufgebracht sein.

An den proximalen Abschnitten der beiden Instrumentenbranchen 4 und 6 wird der Abstand zwischen den Elektrodenflächen 20 vorzugsweise durch ein separates Abstandshaltermodul oder Bauteil 24 bewerkstelligt. Dieses Abstandshaltermodul 24 ist ein Bauteil, das am Schwenkgelenk 17 vorgesehen/drehbar gelagert bzw. Teil des Schwenkgelenks 17 ist und sich zwischen den Instrumentenbranchen 4 und 6 frei um die Schwenkachse A drehen kann. Es ist zwischen zwei Gelenkwangen der oberen Instrumentenbranche 4 aufgenommen. Es weist einen Lagerabschnitt oder Lagerblock auf, an den sich zwei in Richtung hin zu den Branchen 4, 6, d.h. radial von der Schwenkachse A vorspringende flache Materialzungen 28 erstrecken, deren jeweilige Höhe H (Materialdicke) einem zu erzielenden Mindestabstand S zwischen den gegenüberliegenden Elektrodenflächen 20-1 und 20-3 bzw. 20-2 und 20-4 entspricht und deren Quer-Abstand und Breite im Wesentlichen dem Quer-Abstand und der Breite der Elektrodenflächen 20 entsprechen. Das Abstandshaltermodul 24 oder zumindest die Materialzungen 28 sind aus elektrisch nicht leitendem Material.

In der (nicht gezeigten) Schließstellung der Instrumentenbranchen 4 und 6 werden die beiden Materialzungen 28 zwischen den Elektrodenflächen 20-1 und 20-3 bzw. 20-2 und 20-4 eingeklemmt, wodurch der Mindestabstand zwischen den Elektrodenflächen 20-1 und 20-2 einerseits und den Elektrodenflächen 20-3 und 20-4 andererseits am proximalen Ende der Branchen 4, 6 gewahrt bleibt.

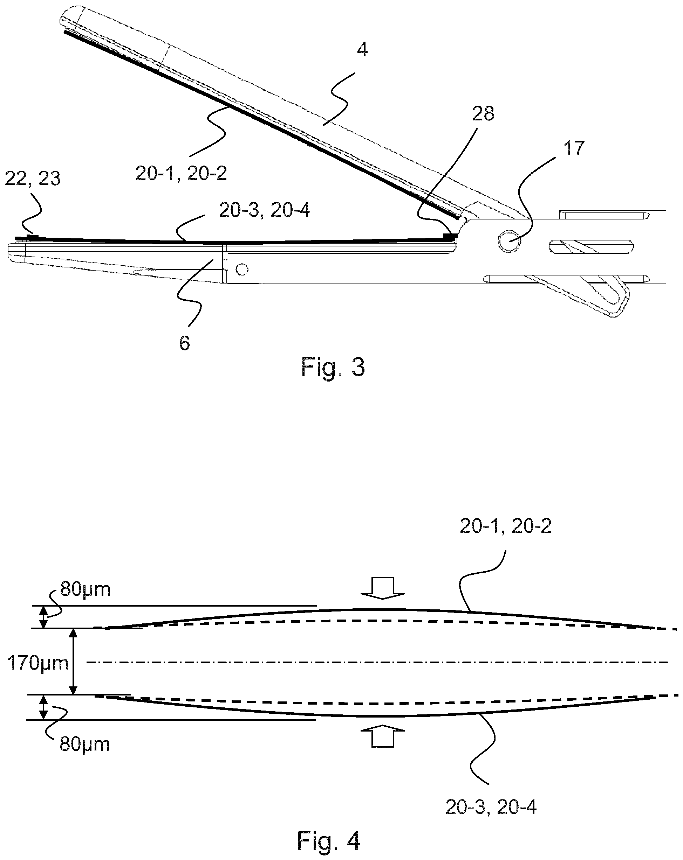

Fig. 3 zeigt eine Seitenansicht der beiden Instrumentenbranchen 4 und 6 der Fig. 2 in einer geöffneten Stellung. Auch wenn nicht sehr deutlich aus der Fig. 3 zu erkennen, so sind die Elektrodenflächen 20-1 und 20-2 der oberen Instrumentenbranche 4 und die Elektrodenflächen 20-3 und 20-4 der unteren Instrumentenbranche 6 über ihre gesamte Längserstreckung jeweils leicht nach außen (die oberen Elektrodenflächen 20-1 und 20-2 nach oben und die unteren Elektrodenflächen 20-3 und 20-4 nach unten) konkav gebogen.

Wenn die Instrumentenbranchen 4 und 6 geschlossen und gegeneinander gedrückt werden, um ein dazwischen eingeklemmtes Gewebe elektrothermisch zu behandeln, kommen, wie bereits vorstehend beschrieben und auch in der Fig. 3 gezeigt, die an den distalen und proximalen Endabschnitten der Instrumentenbranchen 4 und 6 angeordneten und wirkenden Abstandshalter 22, 23 und 24 zuerst in Anlage, weshalb die medialen Abschnitte der Instrumentenbranchen 4 und 6 unter dem auf die Instrumentenbranchen 4 und 6 ausgeübten Anpressdruck mittig gegeneinander durchgebogen werden.

Die konkave bogenförmige Vorspannung/Vorformung der Elektroden bzw. der Elektrodenflächen 20 ist jeweils so gewählt, dass diese der zu erwartenden Durchbiegung in der Schließstellung der Instrumentenbranchen 4 und 6 entspricht, d.h. dass die im unbelasteten Zustand (offenen Stellung der Instrumentenbranchen 4 und 6) leicht konkaven Elektrodenflächen 20 im belasteten Zustand (geschlossene Stellung der Instrumentenbranchen 4 und 6) gerade bzw. plan gebogen werden, d.h. dass die Vorformung der Elektrodenflächen 20 unter der Biegebeanspruchung aufgehoben wird.

Fig. 4 zeigt eine (nicht maßstabsgetreue) Prinzipskizze eines gegenüberliegenden Paars Elektroden 20-1 und 20-3 bzw. 20-2 und 20-4 des elektrochirurgischen Instruments gemäß der ersten Ausführungsform der Erfindung. Die durchgezogenen Linien stellen den Verlauf der Elektrodenflächen im unbelasteten Zustand (offenen Stellung der Instrumentenbranchen 4 und 6 bzw. geschlossenen Stellung ohne Anpressdruck) dar, während die gestrichelten Linien den Verlauf der Elektrodenflächen 20 im belasteten Zustand (geschlossenen Stellung mit Anpressdruck) darstellen.

Aus dieser Skizze ist erkennbar, dass die Elektrodenflächen 20 unter der Biegebeanspruchung, wenn die Instrumentenbranchen 4 und 6 gegeneinander gepresst werden, gerade bzw. plan gebogen werden, so dass die oberen Elektrodenflächen 20-1, 20-2, wenn sich das Gewebe zwischen den Instrumentenbranchen 4 und 6 befindet und die gegenüberliegenden unteren Elektrodenflächen 20-3, 20-4 mit HF-Spannung beaufschlagt werden, die gewünschte parallele Ausrichtung und einen im Wesentlichen gleichmäßigen Abstand zueinander erhalten.

In der Fig. 4 sind ferner Größenordnungen zum einen des gewünschten Mindestabstands der Elektrodenflächen (ca. 170 µm) während der elektrothermischen Behandlung des Gewebes und die Vorbiegung (Bogenhöhe) der Elektrodenflächen der geraden Ausrichtung (ca. 80 µm) angeben, die selbstverständlich je nach Anwendungsfall und Instrumententyp auch abweichen können.

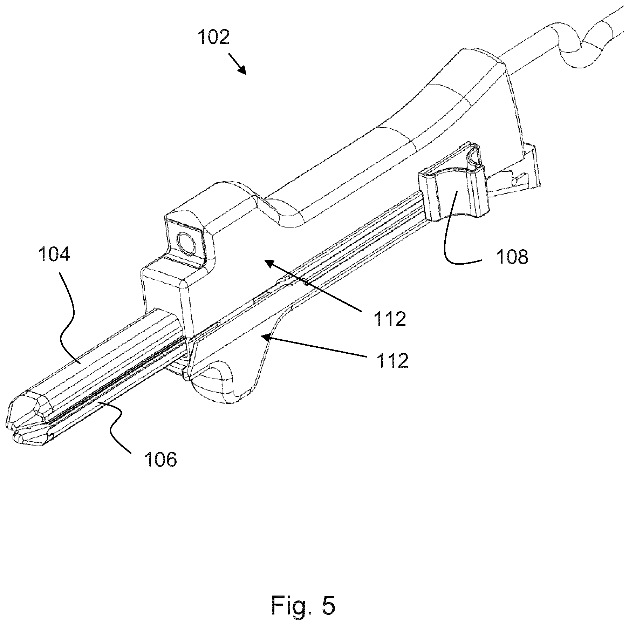

Fig. 5 zeigt ein elektrochirurgischen Instrument 102 gemäß einer zweiten Ausführungsform, welches sich von dem vorstehend beschriebenen Instrument 2 im Instrumententyp unterscheidet. Während die erste Ausführungsform als ein laparaskopisches elektrochirurgisches Instrument 2 mit einem dünnen, langen Schaft 12 und daran schwenkbar angelenkten Instrumentenbranchen 4 und 6 beschrieben wurde, sind die zuvor beschriebenen Abstandshalteranordnungen sowie Elektrodenformen ebenfalls im Zusammenhang mit dem Instrument 102 realisierbar, bei dem die zwei Instrumentenbranchen 104 und 106 gemäß vorstehender Konstruktion über ein manuell betätigbares Schieberelement 108 und einer am Griffstück 112 ausgeformten Gleitmimik gleichzeitig in eine Art Aufnahmeschacht teilweise eingezogen werden, wodurch sich die beiden Branchen 104, 106 automatisch zusammenklappen. Auch in diesem Fall werden in der geschlossenen Stellung gemäß Fig. 5 Biegekräfte auf die Instrumentenbranchen 104 und 106 aufgebracht, welche durch die konkav vorgeformten Elektroden bzw. Elektrodenflächen oder alternativ durch die konkav vorgeformten Branchen kompensiert werden.

Die Erfindung ist nicht auf die zuvor beschriebene Ausführungsformen beschränkt. Diverse Modifikationen innerhalb des Schutzbereichs der beigefügten Ansprüche sind möglich.

Anstelle des Abstandhaltermoduls 24 können ähnlich wie am distalen Ende auch am proximalen Abschnitt der Elektrodenflächen 20 Vorsprüngen auf den Elektrodenflächen aufgebracht sein.

In der beschriebenen ersten Ausführungsform sind die Elektrodenflächen über ihre gesamte Längserstreckung konkav vorgeformt bzw. vorgebogen. Die Elektrodenflächen können jedoch auch lediglich zwischen zwei benachbarten Abstandshaltern konkav gebogen sein.

Ferner können lediglich die Elektrodenflächen 20-3 und 20-4 bzw. Elektroden der unteren Instrumentenbranche 6 bogenförmig vorgespannt sein, da insbesondere die untere Instrumentenbranche 6 mittig angreifenden Biegekräfte ausgesetzt ist, da diese mittig an dem Stützabschnitt 18 wippenförmig gelagert bzw. aufgehängt ist.

Wenn die Instrumentenbranchen in Längserstreckung mehrere Auflagerpunkte oder Abstandshalter haben, können die Elektrodenflächen 20 mehrfach gebogen sein

Die Vorformung der Elektrodenflächen 20, der gesamten Elektrode bzw. der gesamten Instrumentenbranche 4, 6 kann andere Formen als eine konkave Bogenform haben (wie z. B. V-förmig) oder die Vorformung der einzelnen Elektrodenflächen können sich untereinander unterscheiden, solange sichergestellt wird, dass die im belasteten Zustand, d.h. wenn die sich gegenüberliegenden Instrumentenbranchen gegeneinander gedrückt werden, verformten Elektrodenflächen 20 der sich gegenüberliegenden Instrumentenbranchen 4, 6 über deren Längserstreckung einen im Wesentlichen gleichmäßigen Abstand zueinander haben.

Wenn zu erwarten ist, dass in der geschlossenen Stellung der Instrumentenbranchen 4 und 6 die Elektrodenflächen 20 nicht nach innen, sondern nach außen gebogenen werden, können die Elektrodenflächen zumindest abschnittsweise auch nach innen, d.h. konvex, vorgebogen sein.

## Patentansprüche

1. Elektrochirurgisches Instrument (2) mit in einem Schwenkgelenk (17) am distalen Ende eines Instrumentenschafts (12) gegeneinander bewegbaren Instrumentenbranchen (4, 6) mit jeweils einer oder mehreren Elektrodenflächen (20), zwischen welchen ein Gewebe eingeklemmt und elektrothermisch behandelt werden kann, wobei die Bewegung der Instrumentenbranchen (4, 6) relativ zueinander durch zumindest zwei in Längserstreckung der Instrumentenbranchen (4, 6) voneinander beabstandete und auf die Instrumentenbranchen (4, 6) wirkende Abstandshalter (22, 23, 24) begrenzt ist,
**dadurch gekennzeichnet, dass**
zumindest ein in einer Schließstellung der Instrumentenbranchen (4, 6) durch die jeweils gegenüberliegende Instrumentenbranche (4, 6) auf Durchbiegung zu beanspruchender Elektrodenflächenabschnitt, Elektrodenabschnitt oder Branchenabschnitt zumindest einer (6) der Instrumentenbranchen (4, 6) entgegen der in Schließrichtung zu erwartenden Biegerichtung, nämlich in Richtung zur jeweils gegenüberliegenden Instrumentenbranche (4, 6), sowie in Branchenlängsrichtung konkav bogenförmig ausgebildet ist, wobei diese Instrumentenbranche (6) zum Teil in einem schalenförmigen und über das Schwenkgelenk (17) hinaus vorspringenden Stützabschnitt (18) des Instrumentenschafts (12) aufgenommen und mittig an diesem wippenförmig angelenkt ist

2. Elektrochirurgisches Instrument (2) nach Anspruch 1, **dadurch gekennzeichnet, dass**
lediglich ein zwischen den zwei in Längserstreckung der Instrumentenbranchen (4, 6) von einander beabstandeten Abstandshaltern (22, 23, 24) angeordneter Elektrodenflächenabschnitt zumindest dieser Instrumentenbranche (6) bogenförmig ausgebildet ist.

3. Elektrochirurgisches Instrument (2) nach Anspruch 1 oder 2, **dadurch gekennzeichnet, dass**
die Abstandshalter (22, 23, 24) Auflagerpunkte der einander gegenüberliegenden Elektrodenbranchen (4, 6) in Schließstellung der Instrumentenbranchen (4, 6) bilden, welche beim Schließen des Instruments eine vordefinierte Verbiegung des Elektrodenflächenabschnitts, Elektrodenabschnitts oder Branchenabschnitts dieser zumindest einen Instrumentenbranche (6) in Schließrichtung bewirken, wobei der konkav gebogene Elektrodenflächenabschnitt, Elektrodenabschnitt oder Branchenabschnitt dieser zumindest einen Instrumentenbranche (6) entsprechend der in der Schließstellung der Instrumentenbranchen (4, 6) zu erwartenden Biegebeanspruchung sowie der daraus resultierenden vordefinierten Verbiegung des Elektrodenflächenabschnitts, Elektrodenabschnitts oder Branchenabschnitts dieser zumindest einen Instrumentenbranche (6) derart vorgeformt ist, dass der in der Schließstellung der Instrumentenbranchen (4, 6) durch die Abstandshalter (22, 23, 24) definierte Mindestabstand zwischen den Elektrodenflächen (20) nicht unterschritten wird.

4. Elektrochirurgisches Instrument (2) nach Anspruch 3, **dadurch gekennzeichnet, dass**
der Elektrodenflächenabschnitt, Elektrodenabschnitt oder Branchenabschnitt entsprechend der in der Schließstellung der Instrumentenbranchen (4, 6) zu erwartenden Biegebeanspruchung derart vorgeformt ist, dass dieser in der Schließstellung der Instrumentenbranchen (4. 6) im Wesentlichen gerade gebogen wird.

5. Elektrochirurgisches Instrument (2) nach Anspruch 2, **dadurch gekennzeichnet, dass**
der Elektrodenflächenabschnitt entsprechend der Anzahl und der Abstände der Abstandshalter (22, 23, 24) sowie entsprechend dem Elastizitätsmodul der entsprechenden Instrumentenbranche (4, 6) vorgeformt ist.

6. Elektrochirurgisches Instrument (2) nach einem der vorhergehenden Ansprüchen, **dadurch gekennzeichnet, dass**
die Instrumentenbranche (4, 6) samt den Elektrodenflächen (20) bogenförmig ausgebildet ist.

7. Elektrochirurgisches Instrument (2) nach einem der vorhergehenden Ansprüchen, **dadurch gekennzeichnet, dass**
die einander gegenüberliegenden Elektrodenflächen (20-1, 20-3; 20-2, 20-4) der Instrumentenbranchen (4, 6) spiegelsymmetrisch ausgebildet sind.

8. Elektrochirurgisches Instrument (2) nach einem der vorhergehenden Ansprüchen, **dadurch gekennzeichnet, dass**
die zumindest zwei Abstandshalter (22, 23, 24) ausschließlich auf proximale und distale Endabschnitte der Instrumentenbranchen (4, 6) wirken und der mediale Bereich der Elektrodenflächen (20) bogenförmig ausgebildet ist.

## Claims

1. An electrosurgical instrument (2) comprising instrument legs (4, 6) mutually movable in a pivot joint (17) at the distal end of an instrument shaft (12), each instrument leg (4, 6) comprising one or more electrode faces (20) between which tissue can be clamped and treated in an electrothermal manner, the movement of the instrument legs (4, 6) relative to each other being delimited by at least two spacers (22, 23, 24) which are spaced from each other in the longitudinal extension of the instrument legs (4, 6) and act on the instrument legs (4, 6),
**characterized in that**
at least one electrode face portion, electrode portion or leg portion of at least one (6) of the instrument legs (4, 6) to be subjected to deflection in a closed position of the instrument legs (4, 6) by the respective, opposing instrument leg (4, 6) is formed to be concavely bow-shaped as seen in the longitudinal direction of the legs, the curvature being contrary to the bending direction which is to be expected in the closed position, wherein this instrument leg (6) is partially received in a shell-like support/abutment portion (18) of the shaft (12) which projects beyond the pivot joint (17), and is articulated thereto at its center in the manner of a rocker.

2. The electrosurgical instrument (2) according to claim 1, **characterized in that**
only one electrode face portion of at least this instrument leg (6) arranged between the two spacers (22, 23, 24) which are spaced from each other in the longitudinal extension of the instrument legs (4, 6) is formed to be bow-shaped.

3. The electrosurgical instrument (2) according to claim 1 or 2, **characterized in that**
the spacers (22, 23, 24) form support points of the opposing electrode legs (4, 6) in the closing direction of the instrument legs (4, 6), which cause a predefined deformation of the electrode face portion, electrode portion or leg portion of this at least one instrument leg (6) in the closing direction upon closing of the instrument, wherein
the concavely bent electrode face portion, electrode portion or leg portion of this at least one instrument leg (6) portion is preformed according to the bending load to be expected in the closed position of the instrument legs (4, 6) as well as to the resulting, predefined bending of the electrode face portion, electrode portion or leg portion of this at least one instrument leg (6) in such a manner that it does not fall below a minimum spacing between the electrode faces (20) which is defined by the spacers (22, 23, 24) in the closed position of the instrument legs (4, 6).

4. The electrosurgical instrument (2) according to claim 3, **characterized in that**
the electrode face portion, electrode portion or leg portion is preformed according to the bending load to be expected in the closed position of the instrument legs (4, 6) in such a manner that it will be bent so as to be substantially straight in the closed position of the instrument legs (4, 6).

5. The electrosurgical instrument (2) according to claim 2, **characterized in that**
the electrode face portion is preformed according to the number and the distances of the spacers (22, 23, 24) as well as according to the elasticity module of the respective instrument leg (4, 6).

6. The electrosurgical instrument (2) according to one of the preceding claims, **characterized in that**
the instrument legs (4, 6) along with the electrode faces (20) are formed to be bow-shaped.

7. The electrosurgical instrument (2) according to one of the preceding claims, **characterized in that**
the mutually opposing electrode faces (20-1, 20-3; 20-2, 20-4) of the instrument legs (4, 6) are formed to be mirror-symmetric.

8. The electrosurgical instrument (2) according to one of the preceding claims, **characterized in that**
the at least two spacers (22, 23, 24) act exclusively on proximal and distal end portions of the instrument legs (4, 6) and the middle zone of the electrode faces (20) is formed to be bow-shaped.

## Revendications

1. Instrument électrochirurgical (2) avec des branches d'instrument (4, 6) mobiles l'une par rapport à l'autre dans une articulation pivotante (17) au niveau de l'extrémité distale d'une tige d'instrument (12) avec respectivement une ou plusieurs surfaces d'électrode (20), entre lesquelles un tissu peut être serré et traité de manière électrothermique, dans lequel le mouvement des branches d'instrument (4, 6) l'une par rapport à l'autre est limité par au moins deux écarteurs (22, 23, 24) espacés l'un de l'autre dans une direction longitudinale des branches d'instrument (4, 6) et agissant sur les branches d'instrument (4, 6),
**caractérisé en ce que**
au moins une section de surface d'électrode, section d'électrode ou section de branche d'au moins l'une (6) des branches d'instrument (4, 6) à solliciter en flexion dans une position de fermeture des branches d'instrument (4, 6) par les branches d'instruments (4, 6) opposées respectives est réalisée de manière incurvée de manière concave à l'encontre de la direction de flexion attendue dans la direction de fermeture, à savoir dans la direction de la branche d'instrument (4, 6) opposée respectivement, ainsi que dans la direction longitudinale des branches, dans lequel ladite branche d'instrument (6) est reçue en partie dans une section d'appui (18) de la tige d'instrument (12) en forme de cuvette et dépassant à l'extérieur au-dessus de l'articulation pivotante (17) et est articulée avec celle-ci en son centre en forme de bascule.

2. Instrument électrochirurgical (2) selon la revendication 1, **caractérisé en ce que**
seule une section de surface d'électrode disposée entre les deux écarteurs (22, 23, 24) espacés l'un de l'autre dans la direction longitudinale des branches d'instrument (4, 6) au moins de ladite branche d'instrument (6) est réalisée de manière incurvée.

3. Instrument électrochirurgical (2) selon la revendication 1 ou 2, **caractérisé en ce que**
les écarteurs (22, 23, 24) forment des points d'appui des branches d'électrode (4, 6) opposées l'une à l'autre en position de fermeture des branches d'instrument (4, 6) qui, lors de la fermeture de l'instrument, entraînent une flexion prédéfinie de la section de surface d'électrode, section d'électrode ou section de branche de ladite au moins une branche d'instrument (6) dans la direction de fermeture, dans lequel la section de surface d'électrode, section d'électrode ou section de branche de ladite au moins une branche d'instrument (6) fléchie de manière concave est préformée en fonction de la flexion sollicitée attendue dans la position de fermeture des branches d'instruments (4, 6) ainsi que de la flexion prédéfinie qui en résulte de la section de surface d'électrode, section d'électrode ou section de branche de ladite au moins une branche d'instrument (6) de telle sorte que la distance minimale définie entre les surfaces d'électrode (20) dans la position de fermeture des branches d'instruments (4, 6) par les écarteurs (22, 23, 24) ne soit pas dépassée.

4. Instrument électrochirurgical (2) selon la revendication 3, **caractérisé en ce que**
la section de surface d'électrode, section d'électrode ou section de branche correspondant à la flexion sollicitée attendue dans la position de fermeture des branches d'instruments (4, 6) est préformée de telle sorte que celle-ci soit essentiellement droite dans la position de fermeture des branches d'instruments (4, 6).

5. Instrument électrochirurgical (2) selon la revendication 2, **caractérisé en ce que**
la section de surface d'électrode est préformée en fonction du nombre et des distances des écarteurs (22, 23, 24) ainsi qu'en fonction du module d'élasticité des branches d'instrument (4, 6) correspondantes.

6. Instrument électrochirurgical (2) selon l'une quelconque des revendications précédentes, **caractérisé en ce que**
les branches d'instrument (4, 6) ainsi que les surfaces d'électrodes (20) sont réalisées de manière incurvée.

7. Instrument électrochirurgical (2) selon l'une quelconque des revendications précédentes, **caractérisé en ce que**
les surfaces d'électrode (20-1, 20-3; 20-2, 20-4) opposées les unes aux autres des branches d'instrument (4, 6) sont réalisées de manière symétrique.

8. Instrument électrochirurgical (2) selon l'une quelconque des revendications précédentes, **caractérisé en ce que**
les au moins deux écarteurs (22, 23, 24) agissent exclusivement sur des sections d'extrémité proximale et distale des branches d'instruments (4, 6) et la région médiale des surfaces d'électrode (20) est réalisée de manière incurvée.
